# EUROPEAN PATENT SPECIFICATION

(11) **EP 2 667 904 B1**
(45) Date of publication and mention of the grant of the patent: **20.01.2016**
(21) Application number: 12706921.9
(22) Date of filing: 30.01.2012
(51) Int. Cl.: A61L 15/44, A61F 13/08

(54) **COMPRESSION STOCKINGS AND TUBES WITH BIOACTIVE AGENTS FOR THE TREATMENT OF VENOUS INSUFFICIENCY AND VARICOSE VEINS**
KOMPRESSIONSSTRÜMPFE UND SCHLÄUCHE MIT BIOAKTIVEN WIRKSTOFFEN ZUR BEHANDLUNG VON VENENINSUFFIZIENZ UND VARIZEN
TUBES ET BAS DE CONTENTION POURVUS D'AGENTS BIOACTIFS POUR LE TRAITEMENT DE L'INSUFFISANCE VEINEUSE ET DES VARICES

(30) Priority: 28.01.2011 PT 2011105508
(43) Date of publication of application: 04.12.2013
(73) Proprietor: Barcelcom Têxteis S.A., 4750-909 Barcelos (PT)
(72) Inventor: DOS SANTOS MARINHO DA SILVA, Carla Joana, P-4715-343 Braga (PT); DA ROCHA CARDOSO, Ana Patrícia, P-4460-158 Custóias Mts (PT); FERREIRA NETO, Ricardo José, P-4580 Aguiar De Sousa (PT); CAPARRÓS VÁZQUEZ, Cristina Maria, P-4800-010 Guimarães (PT); RIBEIRO PEREIRA, Albina Manuela, P-4710-374 Braga (PT); DE AZEVEDO MIRANDA BAPTISTA, António Lúcio, P-4150-049 Porto (PT); DE SOUSA COUTINHO, Gaspar Augusto Duarte, P-4750 Abade De Neiva (PT)
(74) Representative: Ferreira, Maria Silvina
(86) International application number: PCT/IB2012/050427
(87) International publication number: WO 2012/101618

(56) References cited:
- WO-A2-03/028599
- DE-A1-102004 005 556

## Description

### Technical field of the invention

The present invention describes the production of compression stockings and tubes (knitted on circular machines) containing bioactive agents that are capable of promoting adjuvant beneficial effects on the treatment of vascular diseases, namely the venous insufficiency. In addition to the innovative knitted structure of the compression stockings and tubes, presented in this invention, conferred by the double cylinder circular knitting machines, they have a superficial matrix capable of releasing gradually the functional agents, during the daily usage of the product.

The innovative knitted structure of the stockings and tubes is produced by using a technology developed internally by the inventors, using a double cylinder circular knitting machine, through a system of needles with two heads. The utilization of this knitting technology guarantees the development of stockings and tubes with a higher width and/or length elasticity and allows working with the core spun elasthane fibre in the internal part of stocking. This spun core elasthane fibre is being processed between the loops of the right and reverse side of the knitted structure, without being knitted, which in turns increases the comfort and compression durability of the structure itself. These properties are not present, until now, in similar compression products, that are knitted in mono-cylinder machines.

This innovative knitted structure is covalently linked to an inclusion agent that permits the gradual release of active agents with vascular action from natural, semi-synthetic or synthetic basis. These active principles/agents will be adjuvant on the treatment of the venous system disorders, such as venous insufficiency.

Among the active agents to be included in the compression socks, it can be referred, for example, plants extracts or combinations of plants extracts of horse-chestnut (*Aesculus hippocastanum*), rosemary (*Rosmarinus officinalis*), vine (*Vitis vinifera*), pine (*Pinus maritima*) and butcher's broom (*Ruscus aculeatus*), among others. These extracts will be daily included in the stocking through pulverization, being their skin penetration enhanced by the compression effect of the stocking itself. The gradual release is assured by the presence of inclusion agents in stockings or tubes that will contain the active agents, gradually releasing these active principles for the patient's skin. Among these inclusion agents, it can be referred the α, β and γ-cyclodextrins (CDs).

### Background to the invention

Currently, there is no offer on the market of graduated compression stockings or tubes that contain active agents incorporated in its structure. There are several technologies for producing graduated compression stockings, and several inventions related to the production of socks for the treatment of venous insufficiency are patented. WO/2002/049558 patent concerns the production of therapeutic socks using bi-component fibres with elastomeric interior based on polyurethane and polyamide, in order to provide a good compression. WO/2005/063062 patent reports the production of functional compression stockings with different levels of compression, provided by a knitting technology of multi-stage compression. According to the inventor, these stockings can prevent or even treat varicose veins or oedema.

US/2010/0005568 patent describes a production process of gradient compression hosiery, by using nylon core spun yarns with Lycra® or Coolmax®. According to the inventor, these stockings have compressions that decrease progressively. Despite that, it is not referred in the aforementioned patent the addition of active compounds for the treatment of venous diseases, neither the use of natural cotton fibers, as in the case of the present invention.

On the other hand, the patent EP/1999/0955043 describes the production of garments for cellulite treatment, wherein a anti-cellulite compound is added, that is released into the skin by the action of pH, moisture and/or heat. In this garment, the anti-cellulite compound is linked through ionic linkages (that the inventors claim as semi-durables linkages) to binder agents, including Polyethyleneimine (PEI), which in turn are anchored to the textile material through covalent bonds. According to the inventors, the process for obtaining the substrate in question involves placing nylon in a solution with pH equal to 10.3, together with PEI. They also report that in these conditions, the binder agent PEI adsorbs strongly to nylon by electrostatic interactions and hydrogen bonds, which are not covalent bonds, thus there are no permanent effects. Thus, this present invention differs from the previously described, because it reports compression effects to enhance the releasing of active principles and the use of inclusion agents that are permanently linked to the textile substrate, possessing proven wash fastness to more than 45 washing cycles.

WO/2006/088342 patent describes the production of textile garments, such as socks, gloves, T-shirts, elastic and non-elastic bandages, able to prevent the growth of fungi and bacteria, facilitating the treatment of venous insufficiency, as well as improving the condition of the feet in patients with diabetes. The effect is achieved by using two layers in the article; in the first layer a dye (brilliant green) is adsorbed, and will be removed in the first washing cycle, and in the second layer, silver ions are included. A limitation of this invention is the lack of washfastness for the dye adsorbed in the textile garment (which according to the inventors provides antiseptic activity), as well as the limited colours in which the textile garments can be produced. On the other hand, FR2896808 patent reports the production of an elastic substrate, capable of supplying an active substance to the skin, under the influence of temperature and moisture. The textile substrate is composed of two yarns (core spun yarns), and the spun yarn may contain active ingredients such as seaweed. The patent does not specify or claim the use of any active substances.

Additionally, there are several patents that report the use of plant natural extracts for the treatment of venous insufficiency. WO/2005/120452 patent describes the preparation of creams for the treatment of varicose veins and tired legs, containing extracts of horse-chestnut, rosemary, honey and alcohol as an excipient. Diehm *et al*. (1996) describes a comparative study of the effectiveness (in terms of reduction of oedema) between the use of dry horse-chestnut extract (corresponding to an intake of 50 mg of aescin, twice a day) and compression stockings (class III). This study was conducted in 240 patients with venous insufficiency for 12 weeks. After this period, a reduction in lower leg volume of 43.8 mL (n=95) with the aescin intake and 46.7 mL (n=99) with compression therapy was found, while in the placebo it was observed an increase of 9.8 mL (n=46) after the 12 weeks of therapy. Both therapies have proved to be equivalent, and therefore alternatives and no side effects have been reported. In this study, the two approaches were studied separately and the compression therapy was performed with class III stockings, which means a strong compression 4533-5733 Pa (34-43 mmHg)).

In the present invention it is developed a sock that uses compression stockings class I, characterized by having a soft compression, specifically of 2000-2800 Pa (15-21 mmHg) (and therefore with a lower compression than the stockings reported in the cited article) with a natural extract of horse-chestnut, and also with compression between 2000-4266 Pa (15-32 mmHg), to relieve the symptoms of mild to moderate venous insufficiency.

Compression therapy for the treatment of varicose veins usually involves the use of compression above 2666 Pa (20 mmHg) (stockings belonging to, at least, class II). More severe cases may involve the use of compression stockings with 4533-5733 Pa (34-43 mmHg) (class III) or above 6533 Pa (49 mmHg) (class IV). The stockings with compression less than 20 mmHg are usually well tolerated, by both male and female elements, and therefore have advantages in terms of acceptance by patients, because they are more comfortable, breathable and well tolerated. The problem is that they don't provide effects on treatment of varicose veins (requires compression above 2666 Pa (20 mmHg)) [Cindy e Rooke, 2005].

Thus, in the present invention, the inventors developed, for the first time, a stocking with mild compression, and therefore well tolerated by users, with a natural extract with reported efficacy in combating venous insufficiency, in order to provide the synergism for the treatment of mild to moderate venous insufficiency.

WO/2008/015007 and WO/2010/057856 patents report the use of escin in pharmaceutical preparations for treating diseases caused by allergic shocks or by hypersensitivity reactions. However, there are no reported patents related to escin or other natural plant extracts that are released from inclusion agents immobilized on compression stockings, for the treatment of venous insufficiency.

In the present invention cyclodextrins (CDs) were used as the inclusion agents for the active ingredients with vascular action. Cyclodextrins belongs to the family of cyclic oligosaccharides, resulting from the linkage of several glucose units and have the shape of truncated cones. Its most interesting property is the ability to include within its hydrophobic cavity several molecules (vitamins, colorants, flavourings, essential oils, among other compounds).

Among the various natural cyclodextrins (α-, β-, γ-, corresponding, respectively, to the union of 6, 7, 8 glucose molecules), the β-cyclodextrin expresses the relatively unusual characteristic of exchanging water with the environment, in a quickly and very effective way, which allows to form stable inclusion complexes with a wide variety of molecules. The hydrogen bonds, van der Waals interactions or hydrophobic interactions have been proposed as the interaction forces between the CDs and the encapsulated molecules (FLASHEL ET AL., 1984). Due to its structure, the CDs have the ability to form complexes with compounds in solid, liquid and gaseous states. One of the criteria for the complexation is the size of the molecule to be encapsulated, which must be compatible with the size of the CD's cavity. Additionally, the polarity of the encapsulated molecule and its competition with the other compounds in the environment, also influence the complexation process.

The CDs are among the few complexing agents with unlimited availability and low cost, being usually produced by enzymatic hydrolysis of starch. On the other hand, they have no toxicity and are biologically degradable, being the glucose the main degradation product (ARUNKUMAR *ET AL*., 2005, VIEIRA, 1999).

In the last decade, a great progress was observed in the development of encapsulated substances in CDs, and specifically in the textile area, most of the performed studies concern the encapsulation and release of aromas (BUSCHMANN ET AL., 2001).

However, there are other examples of textile applications of CDs. Cyclodextrins can form complexes with detergent molecules. Thus, they can be considered as a new class of auxiliary substances for the textile industry, with associated advantages, including greater efficiency of detergents and a reduction in the washing water (KNITTEL ET AL., 1992). CDs can be used as surfactants in washing processes for the removal of active agents retained on the fibre surface and can also form complexes in aqueous solution with the dyes used in dyeing processes, which remove the fibre unfixed dyes in the subsequent washing step (CIRELI ET AL., 2006). Other examples include the application of cyclodextrins for easy removal of the sweat (POULAKIS ET AL., 2002; RITTER ET AL., 2002). WO/2002/046520 and WO/2003/042449 patents report a method for the preparation of reactive cyclodextrins, and these reactive cyclodextrins can be incorporated into a textile substrate.

It should be noted that in the mentioned patents it is not referred the washing fastness properties of the textile substrate functionalized with CDs. On the other hand, WO/2007/148678 patent reports the preparation of a textile material for garments that includes CDs. Nevertheless, it is not referred the use of these garments for the treatment of venous diseases, or to incorporate active principles. WO/2006/102728 patent reports the incorporation of flavonoids in cyclodextrins in a pharmaceutical formulation for the treatment of cardiovascular diseases, but there are no patents that report the inclusion of flavonoids in CDs and subsequent binding to a solid substrate.

WO 03/028599 patent discloses compression sleeve for external treatment of the skin of the extremities (legs or arms), comprising a textile flexible hose or stocking which can be tightly pulled over the part of the extremity to be treated. The material of the sleeve is placed in contact with the skin and is used as a support matrix for a microencapsulated chemical, cosmetic or biological substance which can be released from the microcapsules when the areas of the compression sleeve disposed on the skin are rubbed or massaged.

One innovation of the present patent is the fact that the inclusion agent of the active ingredient is covalently bounded to the textile substrate, resisting to more than 30 washing cycles, according to ISO 6330:2000. Thus, the stocking will always be able to receive the active principle, being able to release it gradually to the patient, and therefore, being more effective from the therapeutic point of view. Another important function of the inclusion agent is to direct the active ingredient, so that its penetration into the stratum corneum of the skin is enhanced. The active ingredients that help in the treatment of venous insufficiency, such as escin, are molecules with a hydrophilic and a hydrophobic part. When they are encapsulated into the inclusion agents, they will be oriented with the hydrophilic part toward the outside, and thus, will penetrate more effectively and expeditiously in the skin structure.

### Brief description of the drawings

For a more complete understanding of the invention, a brief description of the drawings is presented. Thus, the figures shown above are referring to:
Figure 1. Atomic force microscopy (AFM) image for the non-functionalized (control) and functionalized stockings, according to the method described in this invention.
Figure 2. Amount of cyclodextrins immobilized in stockings (in %), after several washing cycles in accordance with ISO 6330:2000.
Figura 3. Job plot, showing the formation of complexes between an extract of *Aesculus hippocastanum* and cyclodextrins.

### Summary of the invention

This invention describes the preparation of graduated compression stockings and tubes, containing inclusion agents able to release gradually active ingredients, during the daily usage by the patients, with adjuvant effects in the treatment of venous insufficiency, for example. It is also described in this invention the ability to recharge the active agent in the compression stockings and tubes, so that the oral ingestion of these principles can be replaced. The described stockings and tubes showed a wash fastness higher than 30 washing cycles, so they can be recharged daily, all through their entire life cycle. The tubes of the present invention can be used in stockings, waistbands and sleeves.

Therefore, the object of the present invention is to provide compression tubes, for the treatment of vascular system diseases, comprising the following:
- textile structures with elastomeric properties knitted in double cylinder knitting machines;
- bioactive agents adsorbed into inclusion agents that are covalently linked to the textile structure, forming a superficial matrix capable of releasing gradually the functional bioactive agents.

In a preferable embodiment the compression level of the compression tubes varies between 2000-2666 Pa (15-20 mmHg).

In another preferential embodiment the bioactive agent is natural and preferential are plants extracts or combinations of plants extracts of *Aesculus hippocastanum, Rosmarinus officinalis, Vitis vinifera, Pinus maritima* and *Ruscus aculeatus*.

In another preferential embodiment the bioactive agent is semi-synthetic or synthetic basis, such as diosmin, tribenoside, and calcium dobesilate.

The active ingredients of the compression tubes of the present invention can be recharged by spraying or impregnation, in a solution containing the chosen active ingredient.

In another preferential embodiment the inclusion agents are α, β and γ-cyclodextrins, used in concentrations between 10 and 800g/L, dissolved in NaOH solutions with concentration between 100 and 500 g/L.

In accordance with another preferential embodiment, the textile structures comprise cotton in an amount between 60-95%, elasthane and polyamide.

Another object of the present invention is the method for production of the compression tubes described above that comprises the following steps:
a) knitting of the textile structures (stockings or tubes) in double cylinder knitting machines with fibres;
b) preparation of the functionalization media containing the inclusion agents and crosslinking agents;
c) reaction of the compression stockings with the inclusion agents, via an exhaustion process;
d) washing of the stockings or tubes for removal of the inclusion agents not covalently linked to the substrate;
e) charging the stockings or tubes with the active ingredient.

In a preferential embodiment, the exhaustion process in step b) occur with an alkaline pH, at temperatures between 20 and 80°C under agitation at 100 rpm for 60 minutes, being that the bath ratio can vary between 1:5 and 1:50kg/L.

In another preferential embodiment the washing step c) is made initially with distilled water and, subsequently, with 2g/L of phosphate-free type B at 40°C, for 1 hour.

In another preferential embodiment the crosslinking agents are selected from the group of carbodiimides, amines, aldehydes, and are used in concentrations between 1g/L and 80g/L.

In another preferential embodiment, for the polyamide substrate the permanent fixation of the inclusion agents implies a previous activation, such as acid or alkaline hydrolysis.

Another object of the present invention are the compression stockings comprising the compression tubes described above and obtained by the method described above.

### Detailed description of the invention

According to the present invention, compression stockings or tubes having the capacity to form inclusion complexes with various active ingredients with vascular action (natural and / or synthetic) are prepared. The preparation of stockings or tubes comprises the following steps:
a) Knitting of the textile structure (stockings or tubes) in double cylinder knitting machines with the appropriate fibres;
b) Preparation of the functionalization media containing the inclusion agents and appropriate crosslinking agents;
c) Reaction of the textile structures (compression stockings or tubes) with the inclusion agents, via an exhaustion process, under appropriate pH, temperature and agitation conditions;
d) Washing of the stockings for removal of the inclusion agents not covalently linked to the substrate;
e) Charging the stockings with the active ingredients selected.

In one embodiment of the present invention, the inclusion agents are α, β and γ-cyclodextrins, used in concentrations between 10 and 800g/L, dissolved in NaOH solutions with concentration between 100 and 500 g/L. The bath ratio used on the exhaustion process can vary between 1:5 and 1:50kg/L, and the range of temperatures between 20 and 60°C. The crosslinking agents can be selected from the group of carbodiimides, amines, aldehydes, for example, used in concentrations between 1g/L and 80g/L.

This functionalization process allows the permanent fixation, to the compression stocking, of an amount of cyclodextrins between 0.1 and 10 milligrams of CDs, per gram of compression stocking.

In another embodiment of this invention, the active ingredients is a natural extract of plants, as natural extract of *Aesculus hippocastanum* with proven action in the treatment of venous diseases of the lower limbs, not being this invention however limited to this aspect. The active ingredients may be natural and can be chosen from the group of flavonoids or γ-benzopirones (such as rutin, troxerutin, diosmin and hesperidin, for example), of coumarins and α-benzopirones (benzopirone and its derivatives), of saponins (such as escin, for example), of ergot derivatives (such as dihydroergotamine, dihydroergocristine and diidroergocripitine, for example) or of bioflavonoids. Synthetic active ingredients (or semi-synthetic) can also be used such as diosmin, tribenoside, and calcium dobesilate, for example. Hypothetically, the compression stockings described in this invention may form inclusion complexes with any active ingredient with vascular action, whether this action is phlebotonic/venotonic (promoting vasoconstriction), reduction of capillary permeability or increase of reabsorption.

In another embodiment of this invention, the active ingredients will be charged and recharged by end-user. Based on this new concept, the user can suppress the oral intake of the active ingredient, making the treatment of venous disease expeditious and simple. Thus, the main objective of this invention is to produce stockings able to release an active ingredient for the user's skin with an adjuvant effect in the treatment of venous insufficiency, in a sustained mode, and recharge the active principle whenever there is a medication need.

An immediate advantage of the use of these stockings is to reduce the number of actions that a patient with varicose veins has to execute daily, including the application of a gel with the active ingredient, prior to stockings use. Usually, and by the fact that the gel is applied only once a day, the patient still needs the oral ingestion of the active principle, to ensure the concentrations suitable for the treatment. These new compression stockings will replace these actions, so the only thing patient needs is to wear these stockings with the incorporated active ingredient that will be released at the recommended doses during the day. This procedure, for the simplicity, speediness and the fact that it can eliminate the oral intake of medicines for varicose veins, will have a much greater adhesion by patients suffering from this pathology. The combination of the two approaches of this invention (release and recharge of the active ingredient) allows the development of compression stockings or tubes of high performance, with adjuvant medicinal properties for the treatment of various venous diseases, namely venous insufficiency. As non limiting examples of applications for the stockings described in this invention it can be mentioned: socks to relieve tired legs, for the treatment of venous insufficiency and for preventing economy class syndrome.

### Examples:

The following examples are given only as a means of illustration and should not be considered as limiting the scope of the present invention.

### Example 1

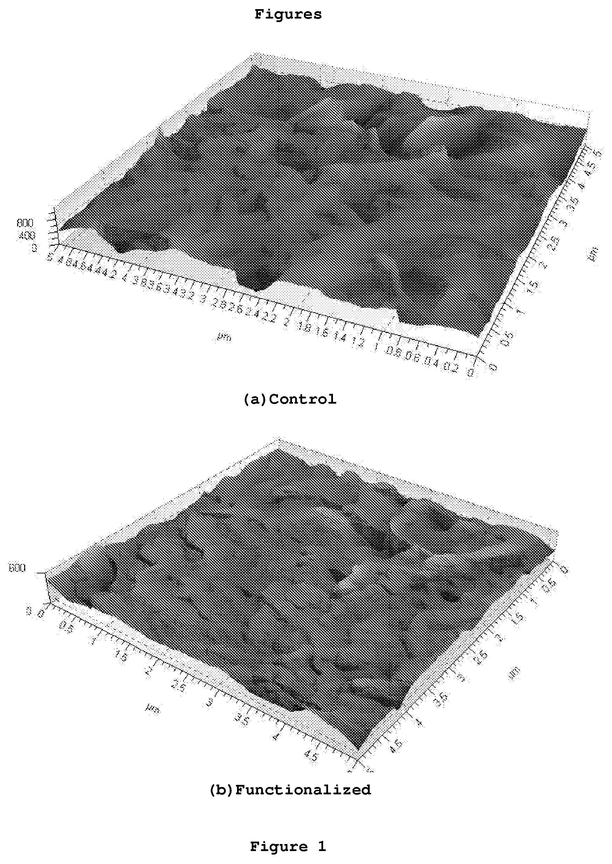

Graduated compression stockings with 72% cotton, 24% polyamide and 4% elasthane core spun with polyamide, knee high, were introduced into a functionalization bath containing cyclodextrins. Cyclodextrins (12.5 g) were dissolved in 1000 mL of NaOH 30%, with 100 rpm agitation at 40°C. After dissolving the cyclodextrins, 4.5 mL of epichlorohydrin was added together with the stockings, using a bath ratio of 1:20 (kg/L). The socks were in the reaction medium for 60 minutes at 40°C with 100 rpm of agitation. After this period of time, the stockings were removed and washed, initially with distilled water and, subsequently, with 2g/L of standard detergent (phosphate-free type B) at 40°C, for 1 hour. Socks were left to air dry. Figure 1 shows the morphological change that occurs on the stocking surface, due to the presence of the inclusion agents.

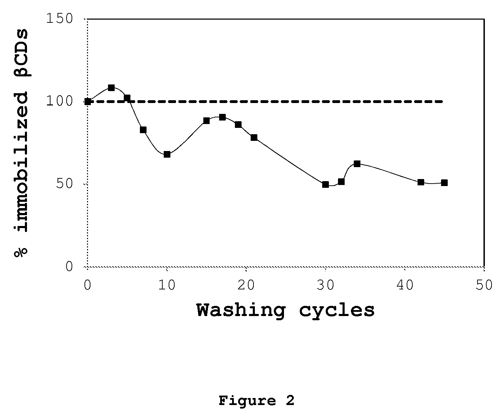

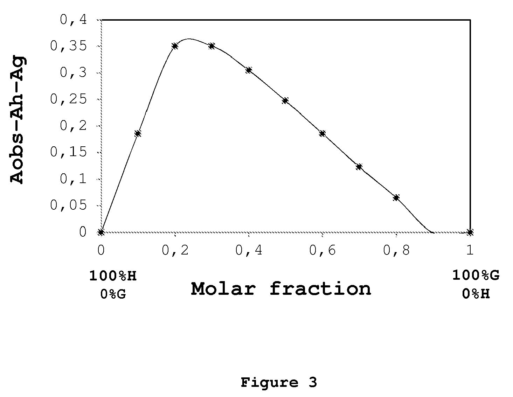

Washing fastness was determined, according to an adaptation of ISO 6330:2000. Thirty washing cycles were carried out on a Mathis Labomat BFA (Werner Mathis AG) equipment, using 4 g/L of standard detergent, without optical brightener, a bath ratio of 1:20 and 20 rpm of agitation, with the addition of one metallic sphere per 100 mL of bath to simulate the mechanical abrasion. Washing cycles were performed at 40°C for 30 minutes (thermal gradient of 3.5°C/min). The functionalized and washed stockings were then tested to quantify the amount of cyclodextrin covalently attached to the stockings, using an internal developed method, based on the complexation with phenolphthalein. Figure 2 evidences this result, showing that the functionalized stockings maintain the same content of cyclodextrins, even after more than 30 washing cycles. The stockings were also sprayed with a solution of 2 g/L of *Aesculus hippocastanum* extract in ethanol at 65%. Figure 3 shows the complexation ability of this extract with the inclusion agents presented in the functionalized stocking. The stockings containing the extract were left to air dry overnight, and afterwards were tested to assess their toxicity, cytotoxicity and potential adverse effect on the skin by *in vivo* corneometry assays. A clinical trial was also carried out in patients with moderate venous insufficiency (CEAP classification 3-4). It has been proved that the stockings obtained by this process had no toxic effects, cytotoxic, nor expressed any kind of allergic reaction in the volunteers of the *in vivo* corneometry study. The clinical trial showed that this compression stocking have a co-adjuvant effect in relieving the symptoms of venous insufficiency. This study also revealed that this type of graduated compression stockings had a high level of satisfaction with regard to comfort, feel and breathability.

### Example 2

Graduated compression stockings with 85% polyamide and 15% elasthane core spun with polyamide, tight high, were introduced into a bath containing sulphuric acid 1M for 15 minutes at 30°C. After this procedure of activation, the stockings were removed, washed with running water and introduced into a functionalization bath containing cyclodextrins. Cyclodextrins (12.5 g) were dissolved in 1000 mL of NaOH 30%, with 100 rpm agitation at 40°C. After dissolving the cyclodextrins, 4.5 mL of epichlorohydrin was added together with the stockings, using a bath ratio of 1:20 (kg/L). Socks were in the reaction medium for 60 minutes at 40°C with 100rpm agitation. After this period of time, the stockings were removed and washed, initially with distilled water and, subsequently, with 2g/L of standard detergent (phosphate-free type B) at 40°C for 1 hour. Then the socks were left to air dry. Figure 1 shows the morphological change that occurs on the stocking surface, due to the presence of the inclusion agents.

Washing fastness was determined, according to an adaptation of ISO 6330:2000. Thirty washing cycles were carried out on a Mathis Labomat BFA (Werner Mathis AG) equipment, using 4g/L of standard detergent, without optical brightener, a bath ratio of 1:20 and 20rpm of agitation, with the addition of one metallic sphere per 100mL of bath to simulate the mechanical abrasion. Washing cycles were performed at 40°C for 30 minutes (thermal gradient of 3.5°C/min). The functionalized and washed stockings were then tested to quantify the amount of cyclodextrin covalently attached to the stockings, using an internal developed method, based on the complexation with phenolphthalein. Figure 2 evidences this result, showing that the functionalized stockings maintain the same content of cyclodextrins, even after more than 30 washing cycles. The stockings were also sprayed with a solution of 2g/L of *Aesculus hippocastanum* extract in ethanol at 65%. Figure 3 shows the complexation ability of this extract with the inclusion agents present in the functionalized stocking. The stockings containing the extract were left to air dry overnight, and afterwards were tested to assess their toxicity, cytotoxicity and potential adverse effect on the skin by *in vivo* corneometry assays. A clinical trial was also carried out in patients with moderate venous insufficiency (CEAP classification 3-4). It has been proved that the stockings obtained by this process had no toxic effects, cytotoxic, nor expressed any kind of allergic reaction in the volunteers of the *in vivo* corneometry study. The clinical trial showed that this compression stocking have a co-adjuvant effect in relieving the symptoms of venous insufficiency. This study also revealed that this type of graduated compression stockings had a high level of satisfaction with regard to comfort, feel and breathability.

### Bibliography

WO/2002/049558; "THERAPEUTIC STOCKINGS" YAKOPSON, Simon, Myron; 2002

WO/2005/063062; "A FUNCTIONAL COMPRESSION SOCKS"; JEONG, Chang Min; 2005

WO/2006/088342; "TEXTILE ARTICLE FOR THERAPEUTIC USE"; LYASHENKO, Inga; NOUSIAINEN, Pertti; PYYKKO, Ilpo; 2006 FR 2006/2896808; "FIL ELASTIQUE AVEC UN FIL DÉNROULEMENT CONTENANT DES SUBSTANCES ACTIVES"; Helmut Gebuhr; 2006 US/2010/0005568; "GRADIENT COMPRESSION HOSIERY KNITTED USING CORESPUN YARNS"; SMITH, Mark WL, DALBEY Jeffrey C, 2010

EP/1999/0955043; "ANTI-CELLULITE PANTYHOSE"; PUGLIESE Peter T, 1999.

WO/2007/148678; "FIBER MATERIAL CONTAINING POLYROTAXANE AND FIBER, AND METHOD FOR PRODUCING THE SAME"; ZHAO C. 2007 WO/2005/120452; "A PROCEDURE IN PRODUCTION OF A CURATIVE CREAMS FOR REGENERATION OF SKIN TISSUE, EASER BREATHING, PSORIASIS, PAINS AND TIREDNESS IN LEGS"; PAVLOVIC, Katica; 2005

WO/2008/015007; "USE OF ESCIN"; GRASSAUER, Andreas; PRIESCHL, Eva; 2008

WO/2010/057856; "USE OF ESCIN FOR TREATMENT OF TYPE IV HYPERSENSITIVITY REACTION"; PRIESCHL-GRASSAUER, Eva; FRIEDRICH, Thomas; 2010

WO/2002/046520; "TEXTILE MATERIAL FINISHED WITH POLYMERIC CYCLODEXTRINS, AND METHOD FOR THE PRODUCTION THEREOF"; BUSCHMANN, Hans-Jürgen; SCHOLLMEYER, Eckhard; 2002

WO/2003/042449; "METHOD FOR PRODUCING REACTIVE CYCLODEXTRINS, TEXTILE MATERIAL PROVIDED WITH SAME, AND USE OF SAID CYCLODEXTRIN DERIVATIVES"; SCHMIDT, Andreas; BUSCHMANN, Hans-Jürgen; KNITTEL, Dierk; SCHOLLMEYER. Eckhard; 2003

WO/2006/102728; "INCLUSION COMPOUNDS OF DIOCLEIN, FLORANOL OR ANALOGS WITH CYCLODEXTRIN AND THEIR USE FOR TREATING CARDIOVASCULAR DISEASES"; SOARES LEMOS, Virginia; DE FRANÇA CORTES, Stayner; ALMEIDA RESENDE, Bruno; LINS GONÇALVES, Roberta; SINISTERRA MILAN, Ruben Dario; SCHMITT, Martine; LUGNIER, Claire; BOURGUIGNON, Jean-Jacques; 2006

Cindy L. Felty, Thom W. Rooke. 2005. Compression Therapy for Chronic Venous Insufficiency. Seminars in vascular surgery. 36-40.

Diehm C, Trampisch HJ, Lange S, Schmidt C. Comparison of leg compression stocking and oral horse-chestnut seed extract therapy in patients with chronic venous insufficiency. Lancet. 1996; 347(8997):292-4.

## Claims

1. Compression tubes, for the treatment of vascular system diseases, comprising:
- textile structures with elastomeric properties knitted in double cylinder knitting machines;
- bioactive agents adsorbed into inclusion agents which are cyclodextrins, that are covalently linked to the textile structure, forming a superficial matrix capable of releasing gradually the functional bioactive agents.

2. Compression tubes, according to claim 1, wherein the compression level varies between 2000-4266 Pa (15-32 mmHg), preferentially between 2000-2800 Pa (15-21 mmHg).

3. Compression tubes, according to any of preceding claims, wherein the bioactive agent is natural, semi-synthetic or synthetic basis.

4. Compression tubes, according to previous claim, wherein the natural active agents are plants extracts or combinations of plants extracts of *Aesculus hippocastanum, Rosmarinus officinalis, Vitis vinifera, Pinus maritima* and *Ruscus aculeatus*.

5. Compression tubes, according to claim 3, wherein the synthetic active agents are as diosmin, tribenoside, and calcium dobesilate.

6. Compression tubes, according to claim 3 wherein the active ingredients can be recharged by spraying or impregnation, in a solution containing the chosen active ingredient.

7. Compression tubes, according to any of the preceding claims, wherein the inclusion agents are α, β and γ-cyclodextrins.

8. Compression tubes, according to any of the preceding claims, wherein the α, β and γ-cyclodextrins are used in concentrations between 10 and 800g/L, dissolved in NaOH solutions with concentration between 100 and 500 g/L.

9. Compression tubes, according to claims 1-9, wherein the textile structures comprise cotton, elasthane and polyamide.

10. Compression tubes, according to any of the preceding claims, which comprises cotton in an amount between 60-95%.

11. A method for production of the compression tubes described in any of the previous claims comprising the following steps:
a) knitting of the tubes in double cylinder knitting machines with fibres;
b) preparation of the functionalization media containing the inclusion agents and crosslinking agents;
c) reaction of the compression tubes with the inclusion agents, via an exhaustion process;
d) washing of the tubes for removal of the inclusion agents not covalently linked to the substrate;
e) charging the tubes with the active ingredient.

12. The method according to any of the previous claim wherein the exhaustion process in step b) occur with an alkaline pH, at temperatures between 20 and 80°C under agitation at 100 rpm for 60 minutes.

13. The method according to claims 11-12 wherein the bath ratio used on the exhaustion process of step b) can vary between 1:5 and 1:50kg/L.

14. The method according to claim 11 wherein the washing step c) is made initially with distilled water and, subsequently, with 2g/L of phosphate-free type B at 40°C, for 1 hour.

15. The method according to claims 11-14 wherein the crosslinking agents are selected from the group of carbodiimides, amines, aldehydes, and are used in concentrations between 1g/L and 80g/L.

16. The method according to claims 11-14 wherein for the polyamide substrate the permanent fixation of the inclusion agents implies a previous activation, such as acid or alkaline hydrolysis.

17. Compression stockings wherein they comprise the compression tubes described in claims 1-10 and obtained by the method described in claims 11-16.

18. Compression sleeves wherein they comprise the compression tubes described in claims 1-10 and obtained by the method described in claims 11-16.

19. Compression waistbands wherein they comprise the compression tubes described in claims 1-10 and obtained by the method described in claims 11-16.

## Patentansprüche

1. Kompressionsröhrchen für die Behandlung von Erkrankungen des Gefässsystems, umfassend:
- in Doppelzylinderstrickmaschinen gestrickte Textilstrukturen mit elastomeren Eigenschaften;
- bioaktive Wirkstoffe, die in Einschlussmittel adsorbiert sind, welche Cyclodextrine sind, die kovalent an die textile Struktur gebunden sind, wodurch eine oberflächliche Matrix gebildet wird, die progressiv die funktionellen bioaktiven Wirkstoffe freisetzt.

2. Kompressionsröhrchen nach Anspruch 1, worin die Kompressionsstufe zwischen 2000 Pa - 4266 Pa (15 mmHg - 32 mmHg), bevorzugt zwischen 2000 Pa - 4800 Pa (15 mmHg - 21 mmHg), variiert.

3. Kompressionsröhrchen nach einem der vorhergehenden Ansprüche, worin der bioaktive Wirkstoff eine natürliche, eine halbsynthetische oder eine synthetische Basis ist.

4. Kompressionsröhrchen nach einem der vorhergehenden Ansprüche, worin die natürlichen aktiven Wirkstoffe Pflanzenextrakte oder Kombinationen von Pflanzenextrakten von *Aesculus hippocastanum*, *Rosmarinus officinalis*, *Vitis vinifera*, *Pinus maritima* und *Ruscus aculeatus* sind.

5. Kompressionsröhrchen nach Anspruch 3, worin die synthetischen aktiven Wirkstoffe Diosmin, Tribenosid und Calcium-Dobesilat sind.

6. Kompressionsröhrchen nach Anspruch 3, worin die aktiven Wirkstoffe durch Aufsprühen oder durch Imprägnieren, in einer Lösung, die den gewählten aktiven Wirkstoff enthält, aufgeladen werden können.

7. Kompressionsröhrchen nach einem der vorhergehenden Ansprüche, worin die Einschlussmittel α-, β- und γ-Cyclodextrine sind.

8. Kompressionsröhrchen nach einem der vorhergehenden Ansprüche, worin die α-, β- und γ-Cyclodextrine in Konzentrationen zwischen 10 g/L und 800 g/L, in NaOH-Lösungen mit Konzentration zwischen 100 g/L und 500 g/L gelöst, eingesetzt werden.

9. Kompressionsröhrchen nach den Ansprüchen 1 - 9, worin die Textilstrukturen Baumwolle, Elasthan und Polyamid umfassen.

10. Kompressionsröhrchen nach einem der vorhergehenden Ansprüche, die Baumwolle in einer Menge zwischen 60% - 95% umfassen.

11. Verfahren zur Herstellung der in einem der vorhergehenden Ansprüche beschriebenen Kompressionsröhrchen, umfassend folgende Schritte:
a) Stricken der Röhrchen in Doppelzylinderstrickmaschinen mit Fasern;
b) Vorbereitung der Funktionalisierungsmedien, die die Einschlussmittel und die Vernetzungsmittel enthalten;
c) Reaktion der Kompressionsröhrchen mit den Einschlussmitteln, über ein Exhaustionsverfahren;
d) Waschen der Röhrchen zur Entfernung der Einschlussmittel, die nicht kovalent an das Substrat gebunden sind;
e) Befüllen der Röhrchen mit dem aktiven Wirkstoff.

12. Das Verfahren nach einem der vorhergehenden Ansprüche, worin das Exhaustionsverfahren in Schritt b) mit einem alkalischen pH-Wert, bei Temperaturen zwischen 20°C und 80°C unter Rühren bei 100 UpM für 60 Minuten, durchgeführt wird.

13. Das Verfahren nach den Ansprüchen 11 - 12, worin das in dem Exhaustionsverfahren in Schritt b) eingesetzte Flottenverhältnis zwischen 1:5 kg/L und 1:50 kg/L variieren kann.

14. Das Verfahren nach Anspruch 11, worin der Waschschritt c) zunächst mit destilliertem Wasser durchgeführt wird und anschließend mit 2 g/L phosphatfreiem Typ B bei 40°C, für 1 Stunde.

15. Das Verfahren nach den Ansprüchen 11 - 14, worin die Vernetzungsmittel aus der Gruppe der Carbodiimide, Amine, Aldehyde ausgewählt werden und in Konzentrationen zwischen 1 g/L und 80 g/L eingesetzt werden.

16. Das Verfahren nach den Ansprüchen 11 - 14, worin für das Polyamidsubstrat die dauerhafte Fixierung der Einschlussmittel eine vorherige Aktivierung impliziert, so wie eine saure oder alkalische Hydrolyse.

17. Kompressionsstrümpfe, worin diese die in den Ansprüchen 1 - 10 beschrieben Kompressionsröhrchen umfassen und durch das in den Ansprüchen 11 - 16 beschriebene Verfahren erhalten werden.

18. Kompressionsmanschetten, worin diese die in den Ansprüchen 1 - 10 beschrieben Kompressionsröhrchen umfassen und durch das in den Ansprüchen 11 - 16 beschriebene Verfahren erhalten werden.

19. Kompressionsgürtel, worin diese die in den Ansprüchen 1 - 10 beschrieben Kompressionsröhrchen umfassen und durch das in den Ansprüchen 11 - 16 beschriebene Verfahren erhalten werden.

## Revendications

1. Tubes de compression, pour le traitement des maladies du système vasculaire, comprenant:
- structures textiles avec des propriétés élastomères tricotées en machines à tricoter à double cylindre;
- agents bioactifs adsorbés dans des agents d'inclusion qui sont liés de manière covalente à la structure textile, en formant une matrice superficielle capable de libérer progressivement les agents bioactifs fonctionnels.

2. Tubes de compression selon la revendication 1, dans lesquels le niveau de compression varie entre 2000 Pa - 4266 Pa (15 mmHg - 32 mmHg), de préférence entre 2000 Pa - 4800 Pa (15 mmHg - 21 mmHg).

3. Tubes de compression selon l'une quelconque des revendications précédentes, dans lesquels l'agent bioactif est une base naturelle, semi-synthétique ou synthétique.

4. Tubes de compression selon l'une quelconque des revendications précédentes, dans lesquels les agents actifs naturels sont des extraits de plantes ou des combinaisons d'extraits de plantes de *Aesculus hippocastanum, Rosmarinus officinalis*, *Vitis vinifera*, *Pinus maritima* et *Ruscus aculeatus*.

5. Tubes de compression selon la revendication 3, dans lesquels les agents actifs synthétiques sont diosmine, tribénoside et dobésilate de calcium.

6. Tubes de compression selon la revendication 3, dans lesquels les ingrédients actifs peuvent être rechargées par pulvérisation ou par imprégnation, dans une solution contenant l'ingrédient actif choisi.

7. Tubes de compression selon l'une quelconque des revendications précédentes, dans lesquels les agents d'inclusion sont les cyclodextrines α, β et γ.

8. Tubes de compression selon l'une quelconque des revendications précédentes, dans lesquels les cyclodextrines α, β et γ sont utilisées à des concentrations entre 10 g/L et 800 g/L, dissoutes dans des solutions de NaOH à une concentration entre 100 g/L et 500 g/L.

9. Tubes de compression selon les revendications 1 - 9, dans lesquels les structures textiles comprennent coton, élasthanne et polyamide.

10. Tubes de compression selon l'une quelconque des revendications précédentes, qui comprennent coton dans une quantité entre 60% - 95%.

11. Un procédé pour la production des tubes de compression selon l'une quelconque des revendications précédentes, comprenant les étapes suivantes:
a) tricotage des tubes dans les machines à tricoter à double cylindre avec des fibres;
b) préparation des moyens de fonctionnalisation contenant les agents d'inclusion et des agents de reticulation;
c) réaction des tubes de compression avec les agents d'inclusion, par l'intermédiaire d'un processus d'épuisement;
d) lavage des tubes pour l'élimination des agents d'inclusion non liés de manière covalente au substrat;
e) chargement des tubes avec l'ingrédient actif.

12. Le procédé selon l'une quelconque des revendications précédentes, dans lequel le procédé d'épuisement à l'étape b) est réalisé à un pH alcalin, à des températures entre 20°C et 80°C sous agitation à 100 rpm pendant 60 minutes.

13. Le procédé selon les revendications 11 - 12, dans lequel le rapport de bain utilisé sur le processus d'épuisement de l'étape b) peut varier entre 1:5 kg/L et 1:50 kg/L.

14. Le procédé selon la revendication 11, dans lequel l'étape de lavage c) est réalisée d'abord avec de l'eau distillée et, par la suite, avec 2 g/L sans phosphate de type B à 40°C, pendant 1 heure.

15. Le procédé selon les revendications 11 - 14, dans lequel les agents de réticulation sont choisis du groupe des carbodiimides, des amines, des aldéhydes, et sont utilisés dans des concentrations entre 1 g/l et 80 g/L.

16. Le procédé selon les revendications 11 - 14, dans lequel pour le substrat de polyamide la fixation permanente des agents d'inclusion implique une activation précédente, telle que l'hydrolyse acide ou alcaline.

17. Bas de compression dans lesquels ils comprennent les tubes de compression décrits dans les revendications 1 - 10 et obtenus par le procédé décrit dans les revendications 11 - 16.

18. Manchons de compression dans lesquels ils comprennent les tubes de compression décrits dans les revendications 1 - 10 et obtenus par le procédé décrit dans les revendications 11 - 16.

19. Ceintures de compression dans lesquelles elles comprennent les tubes de compression décrits dans les revendications 1 - 10 et obtenus par le procédé décrit dans les revendications 11 - 16.
